# EUROPÄISCHE PATENTANMELDUNG

(11) **EP 2 527 321 A1**
(43) Veröffentlichungstag der Anmeldung: **28.11.2012**
(21) Anmeldenummer: 11167853.8
(22) Anmeldetag: 27.05.2011
(51) Int. Cl.: C07C 309/00, C07C 301/00, C09J 4/00, A61K 6/083, C07F 7/18

(54) **Polymerisierbare Verbindungen mit (Meth)Acryloylresten und Sulfonat- oder Sulfatgruppen sowie deren Verwendung**

(71) Anmelder: Fraunhofer-Gesellschaft zur Förderung der angewandten Forschung e.V., 80686 München (DE)
(72) Erfinder: Wolter, Herbert, 97941 Tauberbischofsheim (DE); Seyfried, Mona, 97070 Würzburg (DE); Nique, Somchith, 97249 Eisingen (DE)
(74) Vertreter: Olgemöller, Luitgard Maria

(57) **Zusammenfassung**

Die vorliegende Erfindung betrifft eine Verbindung, umfassend mindestens drei Funktionalitäten, nämlich (a) eine Sulfonat- oder Sulfatgrugruppe, (b) einen (Meth)Acrylrest, sowie (c) entweder (c1) mindestens einen weiteren (Meth)Acrylrest oder eine anorganisch kondensierbare Gruppe, und/oder (c2) eine weitere Carbonsäurefunktion, und/oder (c3) eine Funktion, durch die sich der Brechungsindex eines aus den Verbindungen hergestellten Materials erhöht, nämlich eine Thioethergruppe, mit der Maßgabe, dass in einer siliciumfreien Verbindung eine Sulfonat- oder Sulfatgruppe und ein (Meth)Acrylatrest über einen kohlenwasserstoffhaltigen Rest voneinander getrennt sind, dessen Kohlenstoffkette entweder durch O, S oder NH unterbrochen ist oder eine Verknüpfungsgruppe enthält.

## Beschreibung

Die vorliegende Erfindung betrifft Verbindungen mit mindestens einem (Meth)Acryl-Rest, mindestens einer Sulfonat- oder Sulfatgruppe sowie einer weiteren reaktiven Gruppe, deren Polymerisationsprodukte sowie die Verwendung der Monomeren und der Polymeren im medizinischen Bereich, insbesondere in der Zahnmedizin.

Polymerisierbare, organische Verbindungen mit Säuregruppen sind für medizinische Produkte zum Erreichen gewünschter Materialeigenschaften wie Benetzung, Ätzwirkung, Komplexierung und damit Haftung auf biologischen Grenzflächen wichtige Bestandteile. Dentaladhäsive basieren auf solchen konventionellen, monomeren Verbindungen, weisen allerdings noch einige erhebliche Defizite auf. Ein wesentliches Problem dabei ist, das die Ätzwirkung im Rahmen der Self-etch-Applikation oftmals nicht ausreicht, um die notwendigen retentiven Strukturen zu realisieren, die für die Haftung und damit einen langlebigen Verbund zwischen Zahngewebe und Restaurationsmaterial erforderlich sind. Somit ist oftmals ein vorheriger separater Ätzschritt mit einem Ätzgel nicht vermeidbar, was wiederum die Fehleranfälligkeit und die Behandlungskosten erhöht. Hinsichtlich der zunehmenden Anforderungen hinsichtlich Biokompatibilität (verwiesen sei auf die Allergiediskussion in Verbindung mit dentalen Monomeren) bieten obige Systeme ebenfalls keine Lösung an. Da die Komponenten des Adhäsivs bei einer Restauration den Zahnwurzeln sowie Blutgefäßen am nächsten kommen, ist es aus toxikologischer Sicht von besonderem Interesse, monomerfreie Systeme bereitzustellen.

In der Patentanmeldung DE 44 16 857 C1 werden Carbonsäure-funktionalisierte (Meth)acrylatalkoxysilane beschrieben. Sie zeichnen sich durch eine Vielfalt von Möglichkeiten aus, die Eigenschaften der daraus resultierenden anorganischorganischen Verbundpolymere zu variieren bzw. einzustellen. Bedingt durch die enthaltenen Carbonsäuregruppen ergeben sich zusätzliche Reaktionsmöglichkeiten (z. B. Glasionomerreaktionen) sowie eine verbesserte Haftung auf anorganischen Oberflächen. Die Ätzwirkung (s. Self-etch-Applikation) einer Carbonsäuregruppe ist aber bei weitem nicht so stark, wie es manchmal wünschenswert wäre. Entsprechendes gilt für die in EP 1 377 628 B1 beschriebenen phosphonsäurebasierten Systeme. Mit hybridpolymerbasierten Systemen kann im Rahmen der wünschenswerten Self-etch-Applikationen daher bisher kein ausreichend stabiler Verbund zwischen Zahngewebe und Restaurationsmaterial erzielt werden.

Für eine Reihe von Anwendungszwecken wie die Stabilisierung wässriger Silikate oder die Herstellung von elektroviskosen Flüssigkeiten, Emulgatoren, Detergentien oder Schaumbildnern wurden monomere oder kondensierte Silane entwickelt, die Sulfonat-oder Sulfatgruppen enthalten. So beschreibt US 6,777,521 Siliconsulfat-Polymere, die durch die Umsetzung entsprechender Epoxyverbindungen mit Metallsulfat erhältlich sind. Aus US 3,328,449 sind sulfopropylierte organofunktionelle Silane und Siloxane bekannt, die mit Hilfe der Umsetzung von Sultonen erhalten werden können. US 4,777,277 offenbart Organosiloxan-sulfosuccinate, in denen ein sulfonierter Bernsteinsäureester mit dem Sauerstoffatom der Estergruppe über eine Alkylengruppe an ein Siliciumatom angebunden ist. US 4,503,242 zeigt in Beispiel 1 die Herstellung eines hydrolytisch kondensierbaren Bis-sulfosuccinatamids eines Diaminosilans, erhalten durch die Umsetzung der freien Carbonsäure des entsprechenden Succinatamids mit Natriumsulfit. Ein Silan, das eine Sulfonatgruppe und eine Hydroxygruppe an einem Alkylenoxyalkylenrest des Siliciums trägt, ist in US 5,427,706 offenbart.

Die Verwendung von rein organischen Monomeren, die sowohl eine terminale Sulfonatgruppe als auch eine ungesättigte olefinische Gruppe tragen, für das gleichzeitige Ätzen und Grundieren ("priming") von Zähnen schlägt US 2002/0119426 A1 vor. Das im Beispiel verwendete AMPS (2-Acrylamido-2-methylpropansulfonsäure) ist ein kommerziell erhältliches Produkt. Auch US 6,759,449 B2 offenbart Dentalkleber-Zusammensetzungen, die sowohl eine organisch polymerisierbare (Meth)Acrylsäure-als auch eine saure Gruppe tragen. Dabei wird zwischen Sulfonatgruppen und Phosphonat- oder anderen sauren Gruppen hinsichtlich der Verwendbarkeit der Verbindungen und ihrer Eigenschaften nicht unterschieden. Gleiches gilt für US 2003/0055124 A1; nur für darin gezeigte (Meth)Acrylamido-phosphonsäuren, nicht aber für ebenfalls aufgezeichnete, entsprechende Sulfonsäuren werden Angaben zur Herstellung gemacht. Eine weitere Anmeldung im Wesentlichen derselben Erfindergruppe , US 2008/0194730, schlägt wiederum die Verwendung von selbstätzenden polymerisierbaren N-substituierten (Meth)Acrylsäureamid-Monomeren für Dentalkomposite vor, die zusätzlich eine saure Einheit tragen, ausgewählt unter Phosphonsäure- und Sulfonsäure-Einheiten. N-Methacryloylaminoalkylsulfonsäuren können nach den Erläuterungen der EP 1 421 927 A1 als selbstätzende Primer für dentale Zwecke eingesetzt werden.

Dentalkleber-Zusammensetzungen aus sauer polymerisierbaren Nanoteilen in einer wässrigen Phase offenbart DE 102 06 451 A1. Die Nanoteilchen bestehen aus Siloxanen, an denen sowohl saure als auch organisch polymerisierbare Gruppen gebunden vorliegen. Die sauren Gruppen können entweder Phosphonat- oder Sulfonatgruppen sein; individuelle spezifische Vorteile für die eine oder die andere Gruppe werden nicht genannt. Das einzige Anwendungsbeispiel nennt einen spezifischen Haftungswert eines Dentalklebstoffs aus einem phosphonsäurehaltigen Material an einer Zahnoberfläche. Ein Verfahren zum Herstellen sulfonatgruppenhaltiger Silane oder Siloxane ist weder allgemein noch hinsichtlich der gezeichneten Verbindungen angegeben.

Es besteht ein Bedarf an organisch polymerisierbaren Monomeren mit überlegenen Eigenschaften für die Anwendung insbesondere im Dentalbereich. Besonders relevant sind hier eine verbesserte Haftung und/oder eine verbesserte Ätzfunktion und oder eine Anpassung der optischen Eigenschaften für das kosmetische Erscheinungsbild. Hier Abhilfe zu schaffen, ist die Aufgabe der vorliegenden Erfindung.

In Lösung der Aufgabe werden Verbindungen bereitgestellt, die mindestens drei Funktionalitäten aufweisen, nämlich (a) eine Sulfonsäure- oder Sulfonatgruppe für eine sehr gute Ätzwirkung, (b) einen (Meth)Acrylrest als organisch polymerisierbare Gruppe, durch die das Material nach dem Aufbringen auf oder in den Zahn ausgehärtet werden kann, sowie (c) entweder (c1) mindestens einen weiteren (Meth)Acrylrest oder eine anorganisch kondensierbare Gruppe, um eine besonders gute Vernetzung des Dentalmaterials zu ermöglichen, und/oder (c2) eine weitere Säurefunktion, um die Ätzwirkung des Moleküls zu verbessern, und/oder (c3) eine Funktion, durch die sich der Brechungsindex eines aus den Verbindungen hergestellten Materials erhöht, was zu einer verbesserten Transluzenz und damit einer verbesserten Anpassung an die Zahnfarbe führt. Bei der Säurefunktion gemäß (c2) kann es sich um eine zusätzliche Sulfonsäure- oder Schwefelsäuregruppe handeln; alternativ kann eine andere saure Gruppe vorhanden sein, beispielsweise ein Carbonsäuregruppe.

Den Erfindern ist es gelungen, Wege zur Herstellung von Verbindungen aufzufinden, die sowohl organisch polymerisierbare Gruppen, insbesondere (Meth)Acrylgruppen, als auch Sulfat- oder Sulfonatgruppen aufweisen und die gekennzeichnet sind durch mindestens eine weitere Gruppe, ausgewählt unter einem weiteren (Meth)Acrylrest, einem anorganisch kondensierbaren Silylrest und einer weiteren Säurefunktion. Anstelle der weiteren Gruppe ― oder aber auch zusätzlich ― weisen die Verbindungen eine Thioethergruppe auf, durch die sich der Brechungsindex von aus den Verbindungen hergestellten Dentalmaterialien erhöht.

Die erfindungsgemäßen Verbindungen lassen sich mit der nachstehenden Formel (II) darstellen: 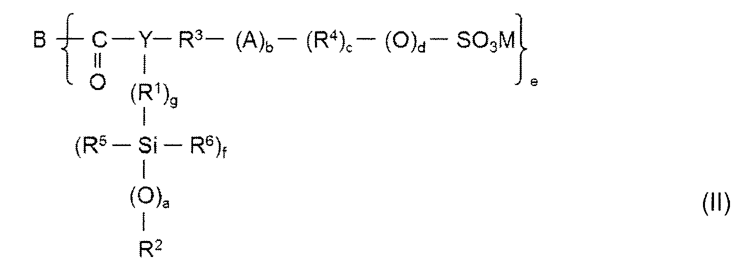 worin
R¹ ein zweibindiger Kohlenwasserstoffrest ist, der, sofern f = 1 ist, über ein Kohlenstoffatom am Siliciumatom gebunden ist,
R² H oder Alkyl ist und zusätzlich im Falle von f =1 und g =1 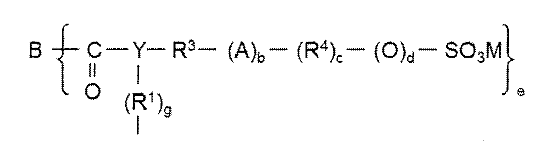 sein kann, wobei in diesem Falle a = 0 ist,
R³ ein, unsubstituiertes oder mit einer funktionellen Gruppe substituiertes, geradkettiges, verzweigtes oder mindestens einen Cyclus aufweisendes Alkylen ist, A eine Verknüpfungsgruppe darstellt,
R⁴ ein gegebenenfalls durch O, S, NH oder NR⁸ unterbrochenes und/oder funktionell substituiertes Alkylen bedeutet,
M Wasserstoff oder ein einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist,
R⁵ und R⁶ unabhängig voneinander entweder unter Hydrolysebedingungen kondensierbare Reste oder substituiertes oder unsubstituiertes, geradkettiges, verzweigtes oder mindestens einen Cyclus aufweisendes Alkyl, Aryl, Arylalkyl, Alkylaryl oder Alkylarylalkyl sind, ausnahmsweise stattdessen aber auch ein entsprechendes Alkylen, Arylalkylen oder Alkylenaryl sein können,
R⁸ C₁-C₆-Alkylen oder ein (Meth)Acrylrest ist,
B Vinyl, 2-Allyl oder, im Falle von e > 1, ein organischer Rest mit e Vinylgruppen ist, die jeweils an eine in der geschweiften Klammer befindliche Gruppe gebunden vorliegen, Y ein Stickstoffatom, -O-CH=, -S-CH= oder -NH-CH= ist, wobei jeweils das Sauerstoffatom, das Schwefelatom bzw. die NH-Gruppe eine Bindung zur benachbarten C(O)-Gruppe aufweist, a = 0 oder 1 ist
b = 0 oder 1 ist
c = 0 oder 1 ist
mit der Maßgabe, dass für die Kombination von Y gleich einem Stickstoffatom, b = 0 und c = 0 der Rest R³ ggf. substituiertes Ethylen ist, sowie der Maßgabe, dass für die Kombination von Y gleich einem Stickstoffatom, b = 0 und c = 1 der Rest R⁴ ein durch O, S, NH oder NR⁸ unterbrochenes und gegebenenfalls funktionell substituiertes Alkylen bedeutet,
d = 0 oder 1 ist, und
e = 1, 2 oder 3 ist
f = 0 oder 1 ist und
g = 0 oder 1 ist,
wobei dann, wenn f = 1 ist, a und g beide # 0 sind und
wobei dann, wenn f = 0 ist, zumindest einer der Reste R³ oder R⁴ einen funktionellen Substituenten trägt, der einen (Meth)Acrylrest oder eine saure Gruppe aufweist oder R⁴ ein mindestens durch S unterbrochenes Alkylen bedeutet.

Wenn sowohl f als auch g = 0 sind, ist a vorzugsweise ebenfalls 0.

Der zweibindige Kohlenwasserstoffrest R¹ kann in allen Ausführungsformen der Erfindung eine Alkylengruppe, eine Arylengruppe oder eine Gruppe sein, die sowohl Alkylen- als auch Aryleneinheiten aufweist. Die Alkylengruppe kann geradkettig oder verzweigt sein und/oder mindestens einen cyclischen Bestandteil besitzen. Wenn g = 1 ist, erfolgt seine Anbindung an das Siliciumatom über ein Kohlenstoffatom. Die Gruppe kann dabei über ein Alkylen-Kohlenstoffatom oder über ein Arylen-Kohlenstoffatom am Siliciumatom gebunden sein. Sie ist unsubstituiert oder unsubstituiert, und sie kann durch ein oder mehrere Sauerstoffatome, Schwefelatome, Estergruppen, Aminogruppen oder Amidgruppen unterbrochen sein.

Die Verknüpfungsgruppe A in der Formel (la) wird vorzugsweise ausgewählt unter (gelesen von links nach rechts in der Formel la) C(O)NH, NHC(O), NR⁸C(O), C(O)O und OC(O), wobei R⁸ wie oben definiert ist. In Ausnahmefällen kann die Verknüpfungsgruppe A jedoch auch zusätzlich unter NHC(O)O, NR⁸C(O)O, NHC(O)NH, C(O)NHC(O) und ―C(O)S- ausgewählt sein. Der Rest R⁴ ist in spezifischen Ausführungsformen substituiert mit mindestens einer Hydroxygruppe und/oder mit einem Rest R⁹COOM, worin R⁹ eine chemische Bindung oder ein C₁-C₆-Alkylenrest ist und M Wasserstoff oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist,

Reste R⁵ und/oder R⁶, die unter Hydrolysebedingungen kondensieren können, werden als anorganische Netzwerkbildner bezeichnet, da sich bei der hydrolytischen Kondensationsreaktion über sie ein Kieselsäurepolykondensat-Netzwerk ausbilden kann. Dem Fachmann ist entsprechend bekannt, welche Bedeutung diese Reste annehmen können. Vorzugsweise sind R⁵ und R⁶ OH oder eine C₁-C₁₀-Alkoxygruppe, stärker bevorzugt eine C₁-C₄-Alkoxygruppe und ganz besonders bevorzugt Methoxy oder Ethoxy. R¹ kann je nach Bedarf aber auch ein Halogenid wie Cl, Wasserstoff, Acyloxy mit vorzugsweise 2 bis 5 Kohlenstoffatomen, Alkylcarbonyl mit vorzugsweise 2 bis 6 Kohlenstoffatomen oder Alkoxycarbonyl mit vorzugsweise 2 bis 6 Kohlenstoffatomen sein.

Der Ausdruck "Sulf(on)at" umfasst die Sulfonat- und die Sulfatgruppe. Die Ausdrücke "Sulfonatgruppe" und "Sulfatgruppe" sollen die jeweiligen Säuren und Salze einschließen.

Das Wort bzw. der Wortbestandteil "(Meth)Acryl..." soll die jeweiligen Methacryl- und Acrylverbindungen gleichermaßen umfassen. Die (Meth)Acrylreste können insbesondere Bestandteil eines (Meth)Acrylsäureesters, -thioesters oder -amids sein. (Meth)Acrylsäureamidreste sind gegenüber den sonstigen (Meth)Acrylresten wegen ihrer besseren Hydrolysebeständigkeit bevorzugt.

Ein Aspekt der Erfindung liegt darin, dass beim Aufbau der chemischen Strukturen der Verbindungen (II) ein bequemer Einbau der (Meth)Acrylgruppen dadurch erfolgen kann, dass diese in Form der freien bzw. einer aktivierten Säure umgesetzt werden. Dies hat zur Folge, dass die (Meth)Acrylgruppe als (Meth)Acrylsäureester, -amid oder―thioester in die Strukturen eingebaut vorliegt.

Mit wenigen Ausnahmen werden die Synthesen so geführt, dass für die Addition der Sulfonsäure- bzw. Sulfatgruppe an das bereits eine (Meth)Acrylgruppe enthaltende Molekül eine C=C-Doppelbindung zur Verfügung steht. Nach Wahl kann an diese entweder Natriumsulfit oder eine Sulfonsäure mit einem bequem addierbaren Rest wie eine Thio- oder Aminoalkansulfonsäure addiert werden. Alternativ kann die Anbindung der Sulfonsäuregruppe auch nach dem umgekehrten Prinzip erfolgen, indem ein Thio-oder Aminoalkylsilan bzw. eine entsprechende silanfreie Verbindung mit einer Alkylensulfonsäure zur Reaktion gebracht wird. Bei diesem Verfahren ist natürlich eine Kettenverlängerung durch die Kohlenstoffatome der Alkylengruppe unvermeidlich, weshalb die erstere Variante gegenüber der zweiten bevorzugt ist. Schließlich gibt es auch noch die Möglichkeit, die Ringöffnung eines reaktiven oder gespannten Heterorings, insbesondere eines Dreirings, mit Sulfit oder einer Hydroxy-, Thio- oder Aminoalkansulfonsäure zu bewirken. Diese Variante hat den Vorteil, dass bei der Ringöffnung eine weitere reaktive Gruppe entsteht, die für die folgende Anbindung der aktivierten (Meth)Acrylsäure genutzt werden kann. Der Heteroring kann alternativ auch mit Hilfe eines Sulfats geöffnet werden; in diesen Fällen erhält man ein sulfatgruppenhaltiges Produkt.

Eine erste Ausgestaltung der Erfindung betrifft Verbindungen mit f = 1 und g = 1, d.h. Silane. Bedingt durch die erfindungsgemäß vorgeschlagenen Herstellungsverfahren werden solche Silane der Formel (II) bevorzugt, in denen dann, wenn eine Sulfonatgruppe über eine durch ein oder mehrere Stickstoffatome unterbrochene Alkylengruppe an das Siliciumatom bzw. an die (ggf. nächstgelegene) (Meth)Acrylgruppe gebunden ist, entweder nicht mehr als zwei Kohlenstoffatome zwischen der Sulfonatgruppe und dem nächstgelegenen Stickstoffatom vorhanden sind oder aber diese Alkylengruppe durch mindestens eine -O-, -S-, -NH-, -C(O)NH-, -NHC(O)- oder-C(O)O- Gruppen unterbrochen ist.

Insgesamt stehen erfindungsgemäß vier grundlegende Varianten für die Herstellung von Silanen mit der Formel (II) mit f = 1 zur Verfügung wie folgt:
Variante (A):
   (a) ein Silan mit einer über ein Kohlenstoffatom an das Si-Atom gebundenen Kohlenwasserstoffgruppe wird bereitgestellt, die mindestens zwei funktionelle Gruppen trägt, ausgewählt unter primären Aminen, sekundären Aminen, Hydroxygruppen und Thiolgruppen,
   (b) eine erste der beiden funktionellen Gruppen wird mit gegebenenfalls aktivierter (Meth)Acrylsäure und die zweite der beiden funktionellen Gruppen wird mit einer gegebenenfalls aktivierten, zweiten, eine C=C-Doppelbindung aufweisenden und gegebenenfalls mindestens eine weitere Funktionalität aufweisenden Carbonsäure umgesetzt, und
   (c) im Anschluss an die genannte Reaktion wird eine sulfonat- oder sulfatgruppenhaltige Verbindung oder ein Sulfit an die C=C-Doppelbindung des mit der zweiten funktionellen Gruppe umgesetzten Carbonsäurerestes addiert, derart, dass an dem mit der ersten der beiden funktionellen Gruppen umgesetzten (Meth)Acrylrest eine solche Addition nicht stattfindet, was sich auf verschiedenen Wegen, z.B. über das molare Verhältnis der miteinander umgesetzten Gruppen, sicherstellen lässt,
      wobei es sich bei der zweiten eine C=C-Doppelbindung aufweisenden Carbonsäure um (Meth)Acrylsäure oder um eine andere doppelbindungshaltige Carbonsäure handeln kann.
Variante (B):
   (a) ein Silan mit einer über ein Kohlenstoffatom an das Si-Atom gebundenen Kohlenwasserstoffgruppe wird bereitgestellt, die mindestens einen reaktionsfähigen Heteroring trägt, ausgewählt unter den Dreiringen Oxacycyclopropyl- (=Epoxy-), Azacyclopropyl und Thiocyclopropyl und cyclischen Carbonaten (letztere können durch Umsetzung eines Epoxyrings mit CO₂, aber auch auf anderen Wegen erhalten werden, wie in DE 44 23811 ausführlich dargestellt),
   (b) der Heteroring wird mit einem Sulfit oder einem Sulfat oder einer sulfonat- oder sulfatgruppenhaltigen Verbindung umgesetzt und
   (c) zumindest die dabei freiwerdende OH-, SH- bzw. NH₂-Gruppe wird mit gegebenenfalls aktivierter (Meth)Acrylsäure umgesetzt.
Variante (C):
   (a) ein Silan mit einer über ein Kohlenstoffatom an das Si-Atom gebundenen Kohlenwasserstoffgruppe wird bereitgestellt, die eine Aminogruppe oder eine Mercaptogruppe aufweist,
   (b) ein Alkenylsulfonat oder ein Sulfon wird mit der Aminogruppe bzw. der Mercaptogruppe zur Reaktion gebracht, und
   (c) die in b. entstandene sekundäre Aminogruppe bzw. Thiogruppe wird mit gegebenenfalls aktivierter (Meth)acrylsäure umgesetzt.
Variante (D):
   a. ein (Meth)Acrylsilan mit mindestens zwei (Meth)Acrylgruppen, die über ein Kohlenstoffatom einer Kohlenwasserstoffgruppe an das Siliciumatom gebunden sind, wird bereit- bzw. nach dem Stand der Technik, z.B. gemäß DE 44 16 857, hergestellt, und
   b. eine sulfonat- oder sulfatgruppenhaltige Verbindung oder ein Sulfit wird im Unterschuss an die C=C-Doppelbindung eines oder mehrerer der (Meth)Acrylreste addiert, derart, dass an mindestens einem (Meth)Acrylrest im Molekül eine solche Addition nicht stattfindet, was sich auf verschiedenen Wegen, z.B. über das molare Verhältnis der miteinander umgesetzten Gruppen, sicherstellen lässt.

In den Varianten (A), (B) und (D) enthält die sulfonat- oder sulfatgruppenhaltige Verbindung vorzugsweise eine OH-, SH- oder NHR¹⁰-Gruppe mit R¹⁰ = Wasserstoff oder C₁-C₆-Alkyl. Beispiele für mercaptogruppenhaltige Sulfonate sind geradkettige oder cyclische Alkansulfonate oder Arylsulfonate, z.B. Mercaptoethansulfonate, Mercaptobutansulfonate, Mercaptocyclohexylsulfonate oder Mercaptobenzolsulfonate. Gängig sind deren Natriumsalze.

Eine zweite Ausgestaltung der Erfindung der Formel (II) betrifft Verbindungen mit f = 0 und g = 0, worin mindestens einer der Reste R³ oder R⁴ einen funktionellen Substituenten trägt, der einen (Meth)Acrylrest und/oder eine saure Gruppe oder ― in manchen Fällen ― stattdessen oder zusätzlich eine Thioethergruppe aufweist. Dabei kann der funktionelle Substituent aus dem (Meth)Acrylrest oder der sauren Gruppe bestehen oder eine diesen Rest/diese Reste umfassende Einheit sein.

Um rein organische Verbindungen herzustellen, können die obigen Varianten (A), (B) und (D) in geeigneter Weise modifiziert werden, indem entsprechende Verbindungen ohne Silylrest als Ausgangsmaterialien eingesetzt werden. Hinsichtlich der Herstellungsvariante (A) ist anzumerken, dass man erfindungsgemäße Verbindungen erhält, wenn an das Ausgangsmaterial mindestens drei doppelbindungshaltige Säuren angebunden werden, was sich z.B. dadurch realisieren lässt, dass eine kohlenwasserstoffhaltige Ausgangsverbindung mindestens drei der in (A)a. genannten funktionellen Gruppen aufweist. Werden an diese drei (Meth)Acrylreste angebunden, können entweder an eine davon oder an zwei davon eine sulfonat- oder sulfatgruppenhaltige Verbindung oder ein Sulfit addiert werden. Erfindungsgemäße Verbindungen erhält man auch, wenn man von einem Ausgangsmaterial mit nur zwei funktionellen Gruppen ausgeht, jedoch mindestens eine davon mit dem Anhydrid einer doppelbindungshaltigen Dicarbonsäure umsetzt. Bei Umsetzungen analog Variante (B) werden dann erfindungsgemäße Verbindungen erhalten, wenn der Heteroring mit einer sulfonat- oder sulfatgruppenhaltigen Verbindung derart umgesetzt wird, dass eine weitere Anknüpfungsstelle für die Anbindung eines zusätzlichen (Meth)Acrylats oder einer weiteren Säure ensteht, d.h. wenn z.B. ein Aminoalkansulfonat mit dem Dreiring umgesetzt wird. In Variante (D) wird anstelle eines (Meth)Acrylsilans eine organische Verbindung mit mindestens drei (Meth)Acrylgruppen eingesetzt, beispielsweise Trimethylolpropantriacrylat. Die schwefelhaltige Verbindung kann dann in einem molaren Verhältnis derart addiert werden, dass das Produkt entweder mindestens zwei (Meth)Acrylreste oder mindestens zwei Sulf(on)atreste enthält. Wenn in den Varianten (A), (B) oder (D) ein Thioalkansulf(on)at verwendet wird, entsteht eine Thioethergruppe, die in manchen Ausführungsformen als dritte funktionelle Gruppe neben dem mindestens einen (Meth)Acrylat und der mindestens einen Sulf(on)atgruppe angesehen werden kann, weil sie den Brechungsindex des daraus herstellbaren Kondensats erhöht, wie oben ausgeführt.

Die verschiedenen Umsetzungen gemäß Variante (A) sollen nachstehend beispielhaft anhand der Reaktionen 1, 2 und 7 näher erläutert werden, wobei die Reaktionen 1 und 2 die Herstellung von Silanverbindungen und Reaktion 7 die Herstellung einer siliciumfreien Verbindung zeigen. Reaktion 7 folgt dabei dem Schema der Reaktion 2.

### Reaktion 1

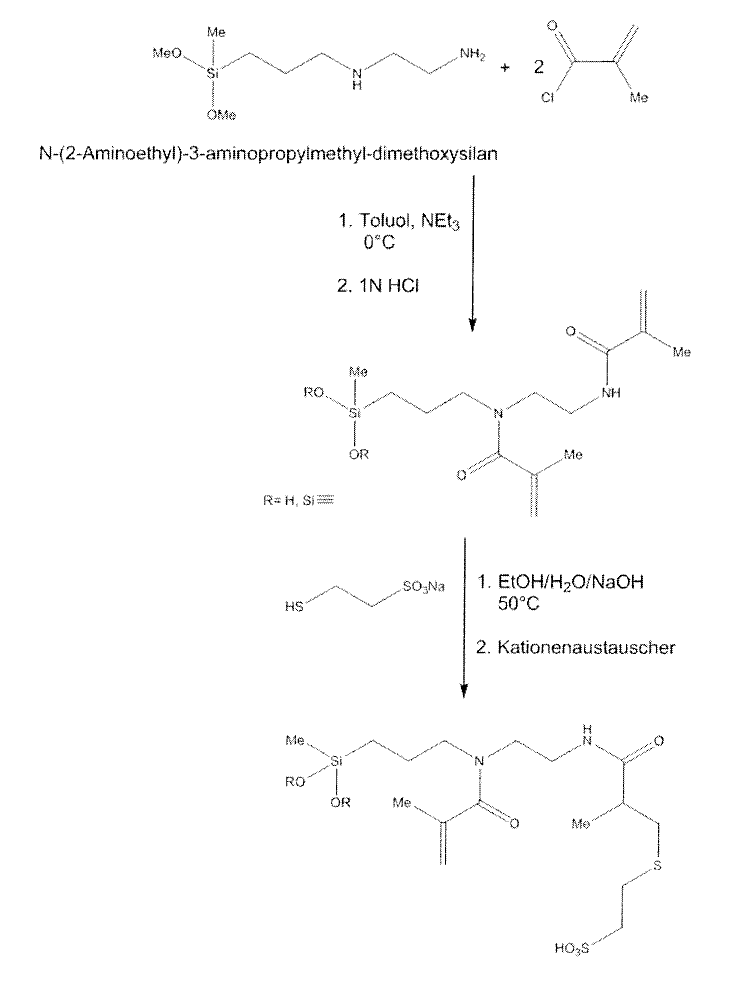

Für Reaktion 1 wurde ein Ausgangssilan mit einer Kohlenwasserstoffgruppe ausgewählt, die zwei Aminofunktionen trägt. Die Reaktion verläuft in vergleichbarer Weise, wenn anstelle des primären, endständigen Amins eine Hydroxy- oder eine Thiolgruppe vorhanden wäre. Die sekundäre NH-Gruppe könnte fehlen; stattdessen könnte die an das Silicium gebundene Alkylengruppe ein durchgehendes Kohlenstoffskelett aufweisen, das an geeigneter Stelle (z.B. an der Position, an der sich im Beispiel die sekundäre NH-Gruppe befindet) mit NH₂, OH oder SH substituiert wäre.

Die Länge und die Struktur der Kohlenwasserstoffgruppe im Molekül ist nicht kritisch und kann beliebig ausgewählt sein. So kann der Kohlenwasserstoff ein geradkettiges oder verzweigtes oder mindestens einen Cyclus aufweisendes Alkyl sein. Außerdem kann der Kohlenwasserstoffl ggf. mit anderen Gruppen substituiert sein, die an den Umsetzungen nicht teilnehmen, oder beliebig durch Heteroatome oder Verknüpfungsgruppen unterbrochen sein.

Die in Reaktion 1 mit Me und OR bezeichneten Substituenten des Siliciums können je nach Bedarf beliebig ausgewählt sein, d.h., es kann eine für das gewünschte Kieselsäure(hetero)polykondensat geeignete Anzahl von hydrolytisch kondensierbaren Resten bzw. von als Netzwerkwandler fungierenden Resten (Alkyl-, Arylgruppen und dgl.) vorhanden sein. In speziellen Ausführungsformen können zwei die erfindungsgemäßen reaktiven Funktionen tragende Kohlenwasserstoffgruppen vorhanden sein; die Summe an hydrolytisch kondensierbaren Resten und als Netzwerkwandler fungierenden Resten beträgt dann 2.

Für die Herstellung des erfindungsgemäßen Silans wird einstufig oder zweistufig eine ausreichende Menge an (aktivierter) (Meth)Acrylsäure bereitgestellt, sodass ein Molekül mit zwei (Meth)Acrylsäureamid-Resten entsteht. Sollte das Ausgangssilan anstelle einer oder beider der Aminogruppen eine oder zwei Hydroxy- bzw. Thiolgruppen enthalten, würden sich jeweils die entsprechenden (Meth)Acrylsäureester- bzw. (Meth)Acrylsäurethioester bilden. Wenn das Ausgangssilan anstelle der sekundären Aminogruppe eine weitere primäre Aminogruppe enthält, entsteht eine Verbindung mit zwei primären (Meth)Acrylamiden.

Im zweiten Schritt der Reaktion wird eine der beiden durch die Anbindung der (Meth)Acrylsäuregruppen ins Molekül eingeführten Doppelbindungen für eine Thiol-en-Addition genutzt. Mit dieser Reaktion wird eine Sulfonsäuregruppe in das Molekül eingeführt. In vergleichbarer Weise lässt sich eine Sulfatgruppe einführen.

### Reaktion 2:

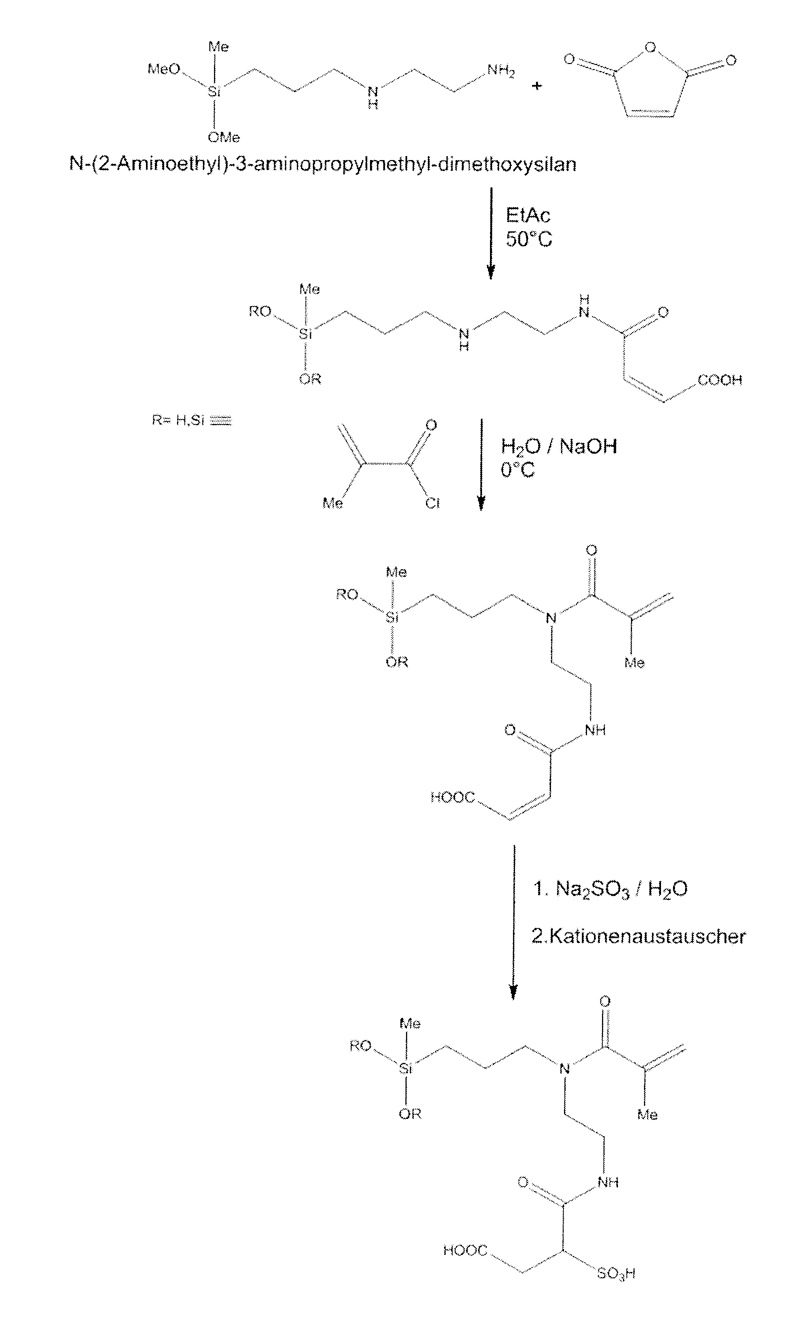

Hinsichtlich der einsetzbaren Ausgangsverbindungen für Reaktion 2 gilt das für Reaktion 1 Gesagte. Die Reaktionsführung unterscheidet sich von Reaktion 1 dadurch, dass in einem ersten Schritt nicht (Meth)Acrylsäure, sondern eine andere, ggf. aktivierte Carbonsäure eingesetzt wird, die eine C=C-Doppelbindung, vorzugsweise in Kombination mit einer C=O-Gruppe (d.h. ein Michael-System, enthaltend die Struktur C=C-C=O) aufweist. Im Beispiel wurde das Anhydrid der Maleinsäure verwendet. Aufgrund der etwas unterschiedlichen Reaktivität der primären und der sekundären Aminogruppen bindet diese, sofern sie nicht im Überschuss eingesetzt wird, in überraschender Weise ausschließlich an die primäre Aminogruppe; die dabei freiwerdende Carbonsäuregruppe ist nicht reaktiv genug, um an der sekundären Aminogruppe anzugreifen, so dass ausschließlich oder fast ausschließlich eine Produkt wie in Reaktion beispielhaft gezeigt entsteht. Dieses wird anschließend mit aktivierter (Meth)Acrylsäure umgesetzt. Mit Hilfe von Natriumsulfit lässt sich schließlich eine Sulfonatgruppe an die Doppelbindung des Maleinsäurerestes addieren, die bei Bedarf in bekannter Weise in eine Sulfonsäuregruppe überführt werden kann.

Das Produkt trägt zusätzlich eine freie Carbonsäuregruppe, die entweder für eine nochmals verbesserte Haft- und Ätzwirkung genutzt oder aber nach Bedarf weiter umgesetzt, z.B. komplexiert werden kann.

### Reaktion 7:

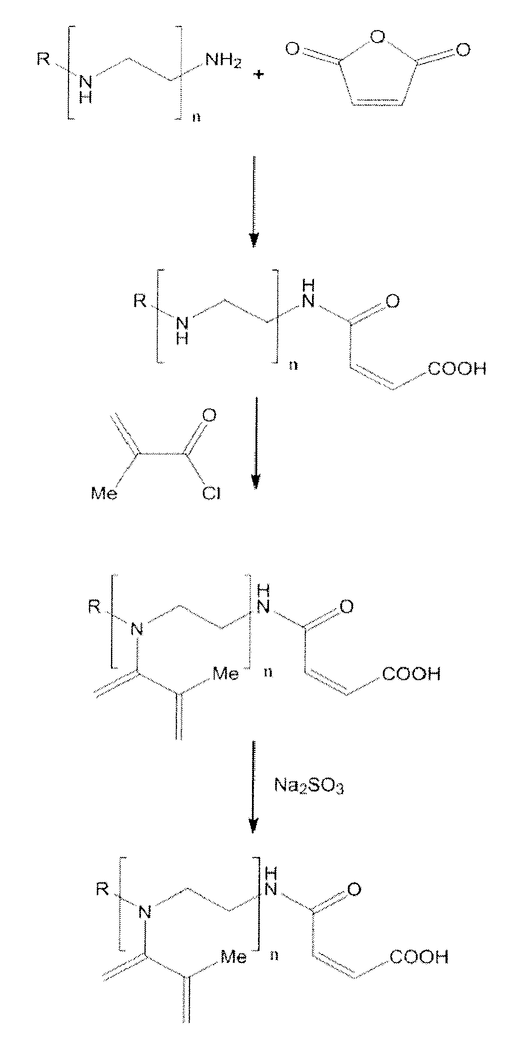

Diese Umsetzung zeichnet sich dadurch besonders aus, dass durch die Auswahl des Index n das Verhältnis von (Meth)Acrylgruppen zu Sulfonsäure- und Carbonsäuregruppen nach Bedarf eingestellt werden kann.

Eine Umsetzung gemäß Variante (B) soll nachstehend beispielhaft anhand der Reaktion 5 näher erläutert werden.

### Reaktion 5a und 5b:

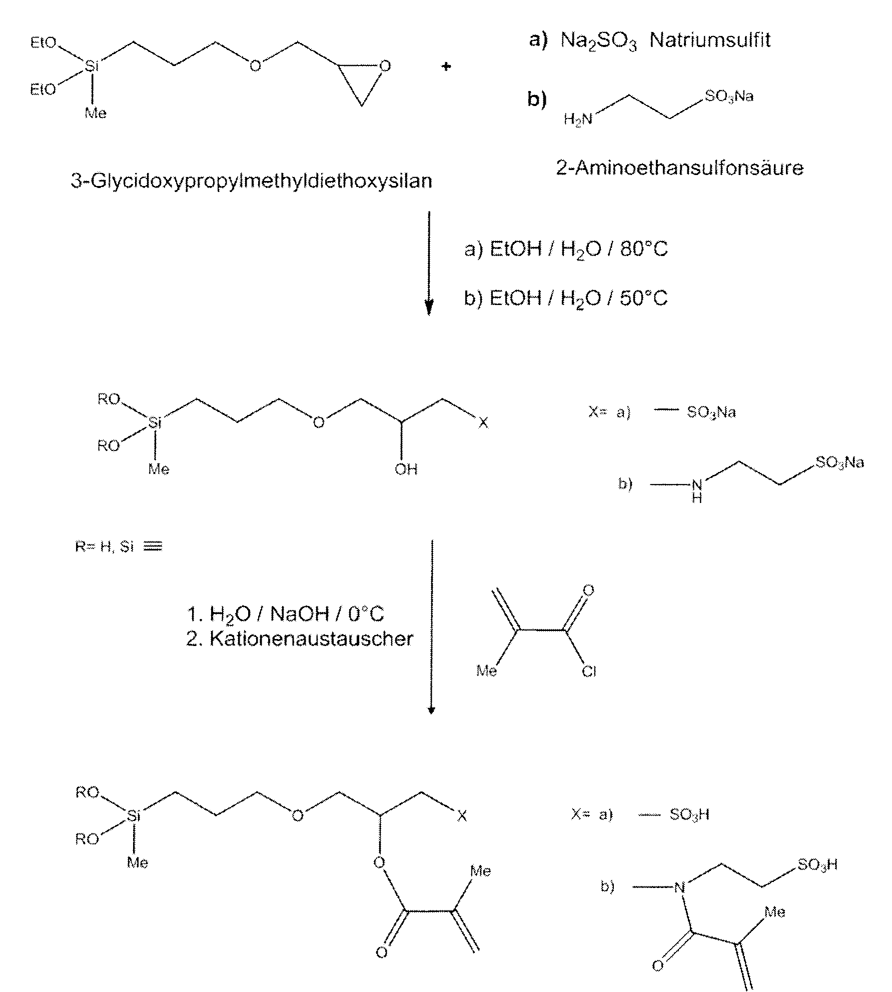

Hinsichtlich der Variabilität des Ausgangssilans sei auf die Ausführungen zu den Reaktionen 1 und 2 verwiesen, die in vergleichbarer Weise auch für die Reaktion 5 gelten. Zwingend ist an der Kohlenwasserstoffgruppe nur das Vorhandensein eines gespannten und damit reaktiven Heterorings. Im gewählten Beispiel ist das eine endständige Epoxygruppe. Die Reaktion ließe sich aber stattdessen auch mit einem Aziridin oder einer Thiocyclopropylgruppe durchführen. Dann würde im ersten Schritt anstelle einer freien Hydroxygruppe eine Amino- oder eine Thiolgruppe entstehen. Wird allerdings anstatt eines gespannten Dreirings ein cyclisches Carbonat eingesetzt, kann die Reaktion nur nach der in der Reaktion 5 gezeigten Variante b) erfolgen.

Wenn der Dreiring direkt mit einem Sulfit oder einem Sulfat umgesetzt wird, ergibt sich zwingend eine Ethylen- bzw. Ethylenoxybrücke zwischen der SO₃Na- oder SO₃H-Gruppe und der Anknüpfungs stelle für die nachfolgende Veresterung der im ersten Schritt entstandenen Hydroxygruppe mit (aktivierter) (Meth)Acrylsäure. Reaktion 5 ist im Übrigen ein Beispiel dafür, dass auch zwei (Meth)Acrylatreste an eine Alkylsilangruppe angekoppelt werden können. Anstelle einer Aminoalkansulfonsäure kann in Reaktion 5 übrigens in allen Fällen auch eine Thio- oder eine Hydroxyalkansulfonsäure verwendet werden. Wird eine Thio- oder Hydroxyalkansulfonsäure eingesetzt, kommt es allerdings nicht zur Anbindung eines zweiten Methacrylatrestes; vielmehr bleibt der Rest, der in der Variante b) über die schlangenlinienförmige Verknüpfung angebunden ist, eine Thio- oder Oxyalkansulfonatgruppe. Auch dann, wenn (Meth)Acrylsäure im Unterschuss verwendet wird, kommt es zur Anbindung nur eines (Meth)Acrylatrestes; die Anbindung erfolgt an der basischsten der vorhandenen Gruppen, d.h. an der einzigen oder der basischsten Aminogruppe, sofern eine solche vorhanden ist.

Wird eine Thioalkansulfonsäure verwendet, entsteht eine Thioethergruppe. Ein Molekül gemäß dem Produkt von Reaktion 5 mit dieser Gruppe, aber ohne den Silylrest, kann unter manchen Aspekten ebenfalls als erfindungsgemäß angesehen werden.

Eine Umsetzung gemäß Variante (C) sollen nachstehend beispielhaft anhand der Reaktion 6 näher erläutert werden.

### Reaktion 6:

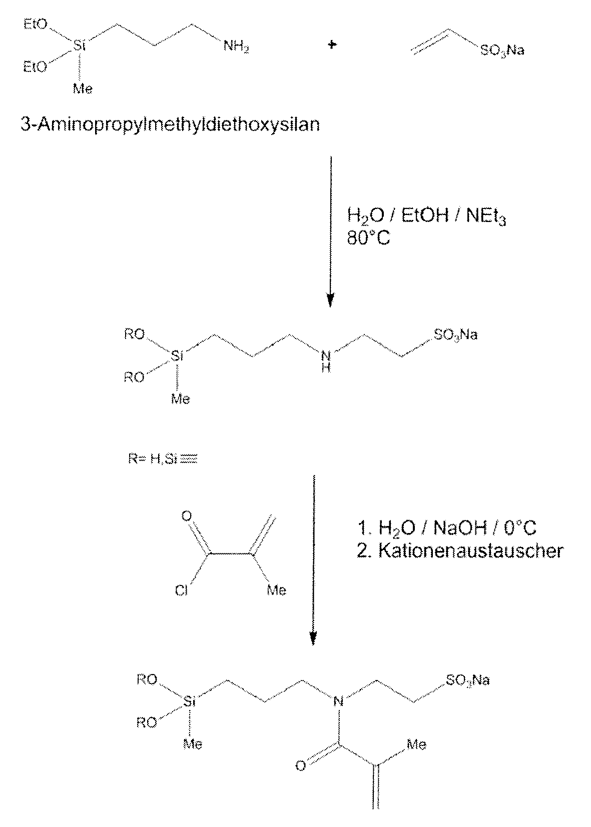

Hinsichtlich der Variabilität des Ausgangssilans sei wiederum auf die Ausführungen zu den Reaktionen 1 und 2 verwiesen, die in vergleichbarer Weise auch für die Reaktion 6 gelten. Die Länge und sonstige Gestalt der Alkylgruppe am Siliciumatom kann frei gewählt werden, sofern sie eine primäre Aminogruppe aufweist. An diese wird erst ein Alkenylsulfonat und anschließend (aktivierte) (Meth)Acrylsäure angekoppelt; das Sulfonat kann wie auch in den übrigen Reaktionsabfolgen anschließend in geeigneter Weise in die freie Sulfonsäuregruppe überführt werden.

Eine Umsetzung gemäß Variante (D) soll nachstehend beispielhaft anhand von Reaktion 4 gezeigt werden:

### Reaktion 4:

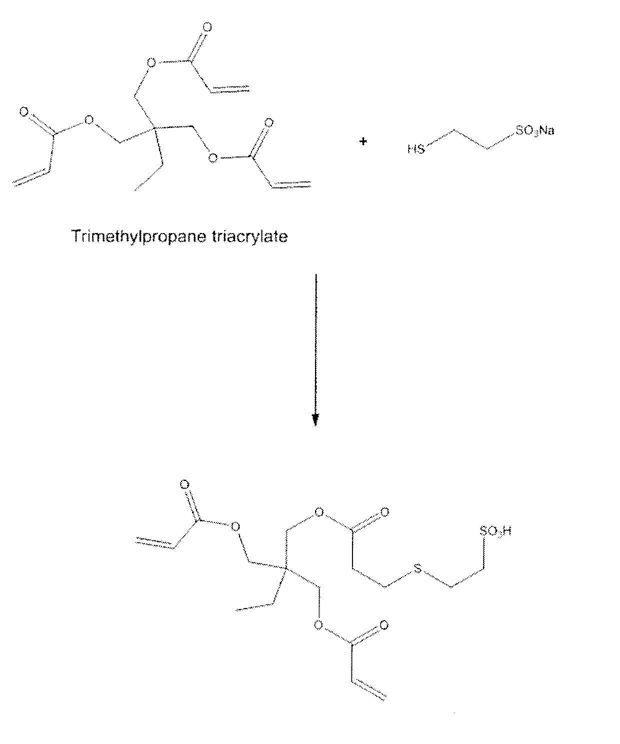

Diese Umsetzung zeichnet sich dadurch aus, dass es durch die Wahl der molaren Menge an Sulfonsäureverbindung möglich ist, Verbindungen mit zwei (Meth)Acrylresten und einem Sulfonsäurerest (bei Einsatz eines Mols Sulfonsäureverbindung pro Mol Methacrylat) oder, wenn die zweifache Molmenge an Sulfonsäureverbindung eingesetzt wird, Verbindungen mit einem (Meth)Acrylrest und zwei Sulfonatresten zu erhalten. Wird eine nichtstöchiometrische Menge an Sulfonat eingesetzt, lassen sich in beliebiger Weise Molekülmischungen erhalten.

Die voranstehenden Reaktionsbeispiele zeigen Umsetzungen zu Sulfonaten. Durch die Verwendung von Sulfaten wie in US 6,777,521 beschrieben anstelle von Sulfiten in den Umsetzungen gemäß Variante (B) lassen sich entsprechende Sulfatverbindungen erhalten.

Soweit es sich bei den erfindungsgemäßen Verbindungen um Silane handelt, können diese durch übliche Methoden (insbesondere das Sol-Gel-Verfahren) hydrolytisch kondensiert werden; es entstehen dabei Kieselsäurepolykondensate, auch als ORMOCER^{®}e bezeichnet. Die Kondensationsreaktion kann in Gegenwart weiterer Silane der Formel SiR*ₐR**₄₋ₐ erfolgen, die aus dem Stand der Technik in sehr großer Zahl bekannt sind. R* bedeutet darin eine hydrolysierbare Gruppe, die das Einkondensieren des Silans in das Netzwerk ermöglicht, während R** ein beliebiger, nicht kondensierbarer Rest ist. Wenn bei der Kondensationsreaktion zusätzlich andere Metallverbindungen vorhanden sein sollen, z.B. Alkoxyverbindungen des Aluminiums, des Titans, des Zirkoniums oder des Zinns, entsteht ein Kieselsäure(hetero)polykondensat, bei dem die genannten Metallatome in das Si-O-Si-Netzwerk eingebunden sind.

Die erfindungsgemäß einsetzbaren Synthesen zeichnen sich durch Einfachheit der Reaktionsführung, eine geringe Anzahl von Arbeitsschritten und gute Ausbeuten aus.

Wie teilweise bereits oben erwähnt, kann in spezifischen Ausführungsformen der Erfindung eine Verbindung mit mehr als einer Sulfonsäure- oder Schwefelsäuregruppe und/oder mit mehr als einer (Meth)Acrylgruppe substituiert sein. Durch das Vorhandensein von mehr als einer (Meth)Acrylgruppe kann das Netzwerk, das sich beim Polymerisieren bildet, noch engmaschiger werden. In diesem Zusammenhang ist zu erwähnen, dass über den Gehalt an polymerisierbaren Doppelbindungen der E-Modul des späteren organisch polymerisierten Polymer eingestellt werden kann, derart, dass das Polymer mehr oder weniger flexibel und dabei weniger hart oder härter ausfällt. Durch das Vorhandensein von mehr als einer Sulfonsäure- oder Schwefelsäuregruppe wird die Ätzwirkung des Materials weiter erhöht.

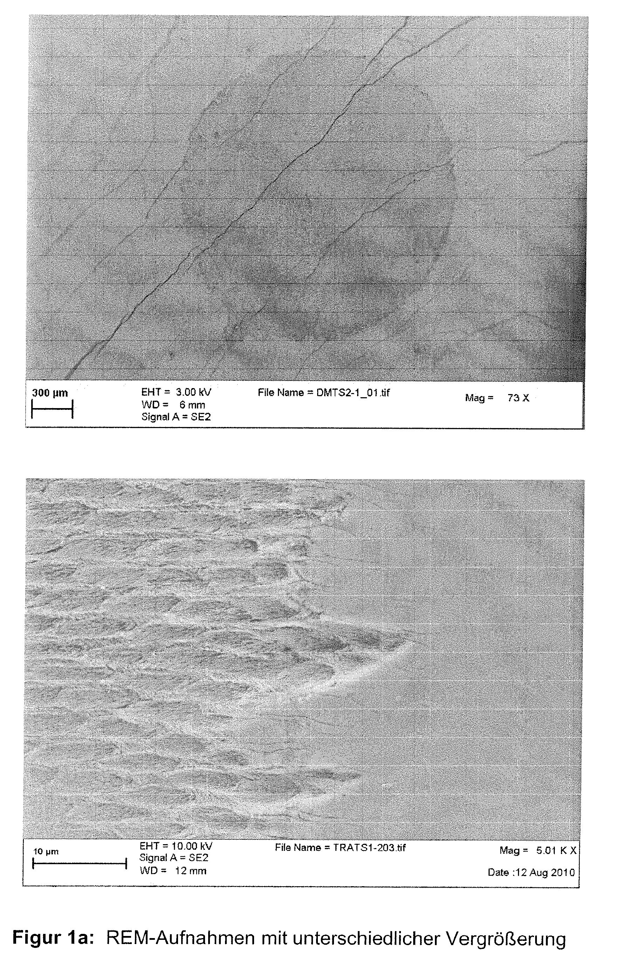

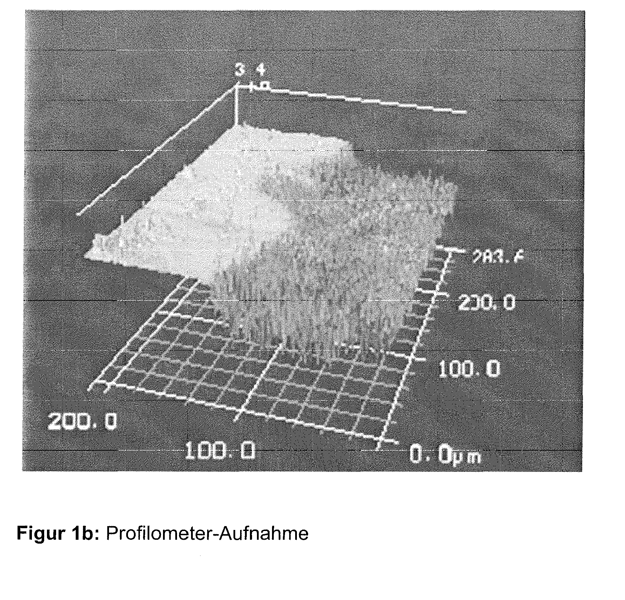

Die Erfinder konnten überraschend feststellen, dass bereits mit geringen Sulfonsäuregehalten eine enorme Ätzwirkung auf dem Zahngewebe beobachtet werden kann Dies lässt sich anhand eines Vergleichs der mittleren Rauheit der Schmelzoberfläche aufzeigen: Polierter Schmelz besitzt eine mittlere Rauheit von etwa 0,21µm, gemessen mit einem optischen Profilometer der Firma UBM. Mit einem phosphonsäurefunktionalisierten Kieselsäurepolykondensat aus Glycerin-1-methacryloyl-2-(siloxypropyl)carboxymethylphosphonsäure kann man Rauheiten im Bereich von 0,33µm erzielen. Mit Kondensaten der erfindungsgemäßen Verbindungen liegt die Rauheit im Bereich von über 0,45µm. Zahnschmelzaufnahmen sind in den Figuren 1a und 1b gezeigt.

Die erfindungsgemäßen Verbindungen, und zwar sowohl die Silane als auch die rein organischen Verbindungen, sind in der Regel wasserlöslich, was in vielerlei Hinsicht vorteilhaft sein kann. Dies mag zu erwarten gewesen sein. Überrascht wurden die Erfinder jedoch davon, dass auch die aus den Silanen erzeugten Kieselsäurepolykondensate in der Regel wasserlöslich sind, obwohl sie ja eine Vielzahl von (Meth)Acrylatgruppen tragen. Dies hat für viele Anwendungen enorme Vorteile, wobei an vorderster Stelle medizinische Applikationen zu nennen sind. Denn die Kondensate können in wässrigem Medium angewendet, d.h. in beliebiger Form appliziert werden, ohne dass der Einsatz eines nichtwässrigen Lösungsmittels erforderlich wäre. Aber auch für industrielle Anwendungen sind wasserbasierte Reaktionen immer vorteilhaft, und zwar schon aus Gründen der Arbeitssicherheit und der Umweltverträglichkeit.

Die Möglichkeit, neben der Sulfonsäurefunktion weitere reaktive Gruppen in den erfindungsgemäßen Verbindungen auszubilden, erschließt zusätzliche Möglichkeiten. So haben Sulfonsäuregruppen eine stärkere Ätzwirkung als Carboxylgruppen, während diese komplexbildende Eigenschaften haben. Sofern zusätzliche Hydroxygruppen vorhanden sind, können diese entweder zur Verbesserung der Benetzung am Untergrund oder für weitere Umsetzungen genutzt werden, die die erfindungsgemäßen Verbindungen weiter modifizieren können. Ein Beispiel ist eine Komplexierung oder eine Umsetzung mit einer Dicarbonsäure (die z.B. mit Hilfe entsprechend aktivierter Säuremoleküle bewirkt werden kann).

Neben Polymerisationsgrad und Ätzwirkung besitzen die an den Verbindungen der Erfindung befindlichen Gruppen und Reste weitere Eigenschaften, die günstig für eine Reihe von Verwendungszwecken sind: Die Sulfonat- bzw. Sulfatgruppe ist ein Ladungsträger, weshalb Verwendungen als Elektrophoresegele, als Materialien für die Elektrotauchlackierung oder als die Leitfähigkeit oder die Antistatik modifizierende Materialien möglich sind. Des Weiteren kann die Gruppe als saurer Katalysator dienen, und zwar zum einen für den Sol-Gel Prozeß im Falle einer hydrolytischen Kondensation erfindungsgemäßer Silane (ein späterer Abtrennungsschritt zur Katalysator-Abtrennung kann dann entfallen) und zum anderen im Hinblick auf den späteren Einsatz (eine Beispiel sind mesoporöse Membranen mit Sulfonsäuregruppen, die als Katalysator für chemische Prozesse dienen können). Die Gruppe sorgt ferner für eine gute Löslichkeit in polaren Medien. Insbesondere, aber nicht nur für dentale Zwecke dient sie als haftvermittelnde Gruppe für anorganische, organische sowie hybride Oberflächen. Genauso wie Carbonsäuregruppen kann sie aber auch ionische Bindungen ausbilden, wodurch sich z.B. Alkali-, Erdalkali-, Ammonium-, Ti-, Zr-, Sn-, Ca- und andere geeignete Kationen in Form ihrer Salze in das Polykondensat-Netzwerk einbinden lassen. Hierdurch lassen sich eine Reihe von Modifikation bzw. materialspezifischen Einstellungen erreichen, z.B. hinsichtlich der Röntgenopazität, der Brechzahl oder der Kontakttoxizität. Die Brechzahl wird darüber hinaus durch das Vorhandensein einer Thioethergruppe beeinflusst (erhöht). Durch die Sulfonat- bzw. Sulfatgruppen im Material erhält dieses im Übrigen eine antimikrobiellen Wirkung. Aber auch auf ganz anderen Gebieten lässt sich die Erfindung anwenden, weil man mit sulfonat- bzw. sulfatgruppenhaltigen Materialien z.B. protonenleitende Membranen, z.B. für fuel cells, bilden kann. Weiter kommen die Materialien in Betracht z.B. als Ionenaustauscher, als pseudostatische Phase in der elektrokinetischen Chromatographie oder als grenzflächenaktiver Stoff (Tensid).

Insbesondere durch die Kombination der Sulfonat- bzw. Sulfatgruppen und ggf. zusätzlich der -CO₂H-Gruppen mit polymerisierbaren / polyaddierbaren Doppelbindungen in einem Molekül bietet sich der Einsatz im Medizinsektor (spez. Dentalbereich) z. B. als Haftvermittler und als Matrixbestandteil für Zemente an. Die Einstellung der Brechzahl kann zur Erhöhung der Transluzenz des Dentalmaterials und damit zu dessen Angleichung an das natürliche Zahnmaterial beitragen.

Mit den erfindungsgemäßen funktionalisierten Verbindungen stehen Verbindungen zur Verfügung, die einerseits direkt verwendet werden können (z.B. für die Funktionalisierung von Oberflächen) und andererseits, sofern es sich um Silane handelt, als Ausgangsverbindungen für die Herstellung von anorganischen Hydrolysaten/Kondensaten (Harzsystemen) sowie von anorganisch / organischen Verbundpolymeren (Matrixsystemen) dienen, d. h. nach organischer Polymerisation/ Polyaddition (Härtung) mit unterschiedlichen Eigenschaften. Mit den silanfreien Monomeren lassen sich rein organische Polymermassen bzw. Polymere erhalten. Durch Einsatz von beliebigen Füllstoffen (Partikel, Fasern) wie z. B. den in DE 1064378, DE 19832965, DE 10018405, DE 1041038 sowie in DE102005018351 beschriebenen Partikeln werden entsprechende Komposite erhalten. Diese plastisch verarbeitbaren Komposite zeichnen sich durch mögliche sehr hohe Füllstoffgehalte (s. Nanohybridkomposite) in Kombination mit einer ausgezeichneten Verarbeitbarkeit aus. Somit können unterschiedliche Eigenschaften in weiten Bereichen sowohl bei den erfindungsgemäßen Verbindungen bzw. Silanen, den daraus erhältlichen Harzsystemen, Matrixsystemen oder rein organischen Polymeren sowie den gefüllten Systemen (Kompositen) eingestellt und den Erfordernissen angepasst werden.

Je nach dem vorgesehenen speziellen Verwendungszweck können dem Kieselsäure(hetero)polykondensat bzw. den organisch zu härtenden Verbindungen geeignete Additive wie Initiatoren, Färbemittel (Farbstoffe oder Pigmente), Oxidationsinhibitoren, Polymerisationsinhibitoren (für die Vermeidung einer vorzeitigen Polymerisation), Verlaufsmittel, UV-Absorber, Stabilisatoren, mikrobiozide Wirkstoffe oder dergleichen zugesetzt werden, wie es dem Fachmann bekannt ist. Beispiele für Polymerisationsinitiatoren sind Initiatoren für die radikalische Polymerisation, und zwar für die thermische Härtung wie Peroxide (z.B. Dibenzoylperoxid) oder Photoinitiatoren wie Benzophenon, Campherchinon oder Kombinationen von α-Diketonen mit Aminen als Reduktionsmittel, wie beispielsweise aus der DE 199 03 177 C2 bekannt. Für die duale Aushärtung von radikalisch und kationisch polymerisierbaren Systemen können insbesondere Diaryliodonium- oder Triarylsulfoniumsalze zugesetzt werden, für die die vorgenannte Druckschrift ebenfalls Beispiele angibt.

Ein gefülltes Dentalkomposit (d.h. ein organisch noch nicht vernetztes, gefülltes Harz aus hydrolysierten und kondensierten Silanen der Erfindung) kann, nachdem es für den vorgesehenen Zweck angewendet wurde, in geeigneter Weise organisch vernetzt und damit ausgehärtet werden. Dem dient vor allem eine organische Polymerisation der (Meth-)Acrylatgruppen. Auch die silanfreien Verbindungen können über eine Polymerisation dieser Gruppen in ein Polymer überführt werden. Dies ist eine radikalische Polymerisation, die üblicherweise unter Zusatz von Radikalstartern wie den oben erwähnten und ggf. bekannten Aktivatoren unter Belichtung mit z. B. sichtbarem Licht (Blaulicht; Dentalstrahler) d. h. photochemisch, thermisch oder redoxinduziert, aber auch im Rahmen von 2-Komponenten-Reaktionen oder anaerob erfolgt. Die Kombination von Selbsthärtung mit z. B, photoinduzierter bzw. thermischer Härtung ist ebenfalls möglich.

Der Einsatz solcher Materialien erstreckt sich u. a. auf die Verwendung in Form von Bulkmaterialien, Kompositen, Zementen, Klebstoffen, Vergussmassen, Beschichtungsmaterialien, Haftvermittlern, Bindemitteln für keramische Partikel (keramische Formgebungsverfahren), zur Herstellung bzw. Primung von Füllstoffen und Fasern, Einsatz im Reaktionsextruder und dergleichen für die verschiedensten Zwecke (insbesondere für medizinische-, aber auch für (mikro)optische- und (mikro)elektronische Anwendungen).

Nachstehend werden Herstellungsverfahren für die obigen Reaktionen beispielhaft angegeben.

### Reaktion 1:

Stufe 1: 5.11 g (0.024 mol) N-(2-Aminoethyl)-3-aminopropylmethyldimethoxysilan wurde in 5.21 g Triethylamin und 30 mL Toluol gelöst und auf 0°C temperiert. Anschließend wurden 5.0 mL (0.051 mol) Methacrylsäurechlorid in 30 ml Toluol hinzu getropft. Das Reaktionsgemisch wurde 3 h bei Raumtemperatur gerührt. Das Gemisch wurde zentrifugiert und die erhaltene Lösung zur Hydrolyse und Kondensation mit 1 N Salzsäure auf pH 1-2 eingestellt. Nach 24 h wurden die flüchtigen Bestandteile unter Vakuum entfernt
Stufe 2: 3.92 g (0.013 mol) des Produkts aus Stufe 1 wurden in 30 mL Ethanol gelöst, die Lösung mit Natriumhydroxid auf pH 10 eingestellt und auf 60°C erwärmt. Anschließend wurden 1.93 g (0.015 mol) Natrium 2-Mercaptoethansulfonat gelöst in 40 mL H₂O zugetropft und für 4 h gerührt. Ethanol wurde unter Vakuum entfernt und die wässrige Lösung mit einem Kationenaustauscher behandelt. Die flüchtigen Bestandteile wurden unter Vakuum entfernt. Das Endprodukt ist in Wasser wieder löslich.

### Reaktion 2:

Stufe 1: 8.69 g (0.042 mol) N-(2-Aminoethyl)-3-aminopropylmethyldimethoxysilan wurden in 50 mL Ethylacetat gelöst und auf 50°C erwärmt. Eine Lösung aus 4.23 g (0.043 mol) Maleinsäureanhydrid in 30 mL Ethylacetat wurde zugetropft und 19 h gerührt. Das Gemisch wurde zentrifugiert und der Rückstand zweimal mit Ethylacetat gereinigt und unter Vakuum getrocknet.
Stufe 2: 6.06 g (0.021 mol) des Produkts der Stufe 1 wurden in 5 mL Wasser und 1.72 g NaOH gelöst und auf 0°C temperiert. 2.1 mL (0.021 mol) Methacrylsäure-chlorid wurden unter starkem Rühren langsam hinzu getropft und für 5 h bei 50°C gerührt. Die flüchtigen Bestandteile wurden anschließend unter Vakuum entfernt.
Stufe 3: 9.82 g (0.021 mol) des Produkts der Stufe 2 wurden in 20 mL Wasser gelöst und auf 60° erwärmt. Anschließend wurden 2.61 g (0.021 mol) Natriumsulfit unter Rühren zugetropft und für 24 h gerührt. Die wässrige Lösung wurde mit einem Kationenaustauscher behandelt und die flüchtigen Bestandteile unter Vakuum entfernt. Das Produkt lässt sich in Wasser wieder lösen.

### Reaktion 5:

a)
   Stufe 1: In 30 mL H₂O wurden 5.04 g (0.040 mol) Natriumsulfit gelöst und auf 80°C erhitzt. Eine Lösung aus 9.96 g (0.040 mol) 3-Glycidoxypropylmethyldiethoxysilan in 10 mL Ethanol wurde hinzu getropft und für 3 h unter Rückfluss gerührt. Nach Abdampfen von Ethanol wurde die wässrige Phase mit Ethylacetat gereinigt und die flüchtigen Bestandteile anschließend unter Vakuum entfernt.
   Stufe 2: 5.04 g (0.016 mol) des Produkts der Stufe 1 wurde in 10 mL Wasser und 2.79 g (0.070 mol) NaOH gelöst und auf 0°C temperiert. 4.0 mL (0.016 mol) Methacrylsäurechlorid wurden anschließend zugetropft und das Reaktionsgemisch für 4 h bei 30°C gerührt. Die Lösung wurde mit Ethylacetat gereinigt, die wässrige Phase mit einem Kationenaustauscher behandelt und die flüchtigen Bestandteile anschließend unter Vakuum entfernt. Das Produkt lässt sich in Wasser wieder lösen.
b)
   Stufe 1: 3.38 g (0.027 mol) 2-Aminoethansulfonsäure wurden in 40 mL H₂O gelöst und mit 1 N NaOH-Lösung auf pH 14 eingestellt. Eine Lösung aus 6.81 g (0.027 mol) 3-Glycidoxypropylmethyldiethoxysilan in 30 mL Ethanol wurden bei 50°C zugetropft und für 3 h gerührt. Danach wurde Ethanol unter Vakuum entfernt und die wässrige Lösung mit Ethylacetat gereinigt. Die flüchtigen Komponenten wurden anschließend unter Vakuum entfernt.
   Stufe 2: 5.12 g (0.014 mol) des Produkts der Stufe 1 wurde in 10 mL Wasser und 2.58 g (0.065 mol) NaOH gelöst und auf 0°C temperiert. 1.5 mL (0.016 mol) Methacrylsäurechlorid wurden zugetropft und das Reaktionsgemisch für 4 h bei 30°C gerührt. Die wässrige Lösung wurde mit Ethylacetat gereinigt und mit einem Kationenaustauscher behandelt. Die flüchtigen Komponenten wurden anschließend unter Vakuum entfernt. Das Produkt lässt sich in Wasser wieder lösen.

### Reaktion 6:

Stufe 1: 5.14 g (0.027 mol) 3-Aminopropylmethyldiethoxysilan wurden in 30 mL Ethanol und 1.65 g (0.016 g) Triethylamin gelöst und auf 70°C erhitzt. Anschließend wurden 14 mL (0.027 mol) einer 25% wässrigen Natriumvinylsulfonat-Lösung zugetropft und für 24 h gerührt. Ethanol wurde anschließend unter Vakuum entfernt und die wässrige Lösung mit Ethylacetat gewaschen. Die flüchtigen Komponenten wurden anschließend unter Vakuum entfernt.
Stufe 2: 5.73 g (0.018 mol) des Produkts der Stufe 1 wurde in wurde in 10 mL Wasser und 2.92 g (0.075 mol) NaOH gelöst und auf 0°C temperiert. 1.8 mL (0.018 mol) Methacrylsäurechlorid wurden zugetropft und das Reaktionsgemisch für 5 h bei 30° gerührt. Das Lösungsmittel wurde anschließend unter Vakuum entfernt. Die wässrige Lösung wurde mit Ethylacetat gereinigt und mit einem Kationenaustauscher behandelt. Die flüchtigen Komponenten wurden anschließend unter Vakuum entfernt. Das Produkt lässt sich in Wasser wieder lösen.

### Reaktion 4:

1.50 g (0.005 mol) Trimethylpropantriacrylat und 13.9 mg (0.06 Gew.%) Butylhydroxytoluol wurden in 20 mL Ethanol gelöst und auf 40°C erwärmt. 0.87 g (0.005 mol) Natrium 2-Mercaptoethansulfonat wurden in 20 mL Wasser gelöst und zugetropft. Das Reaktionsgemisch wurde 4 h gerührt und Ethanol anschließend bei 40°C unter Vakuum entfernt. Die wässrige Lösung wurde danach mit Ethylacetat gereinigt, mit einem Kationenaustauscher behandelt und die flüchtigen Bestandteile unter Vakuum entfernt. Das Produkt kann wieder in Wasser gelöst werden.

## Patentansprüche

**1.** Verbindung, umfassend mindestens drei Funktionalitäten, nämlich (a) eine Sulfonat- oder Sulfatgrugruppe, (b) einen (Meth)Acrylrest, sowie (c) entweder (c1) mindestens einen weiteren (Meth)Acrylrest oder eine anorganisch kondensierbare Gruppe, und/oder (c2) eine weitere Carbonsäurefunktion, und/oder (c3) eine Funktion, durch die sich der Brechungsindex eines aus den Verbindungen hergestellten Materials erhöht, nämlich eine Thioethergruppe, mit der Maßgabe, dass in einer siliciumfreien Verbindung eine Sulfonat- oder Sulfatgruppe und ein (Meth)Acrylatrest über einen kohlenwasserstoffhaltigen Rest voneinander getrennt sind, dessen Kohlenstoffkette entweder durch O, S oder NH unterbrochen ist oder eine Verknüpfungsgruppe enthält.

**2.** Verbindung nach Anspruch 1 mit der Formel (II) 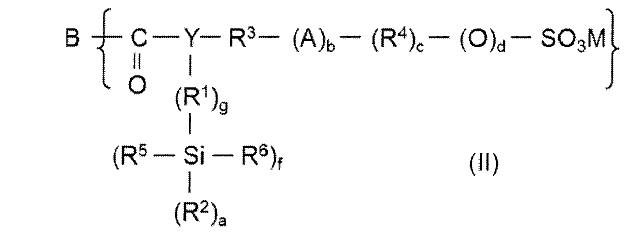 worin
R¹ ein zweibindiger Kohlenwasserstoffrest ist, der, sofern f = 1 ist, über ein Kohlenstoffatom am Siliciumatom gebunden ist,
R² H oder Alkyl ist und zusätzlich im Falle von f =1 und g =1 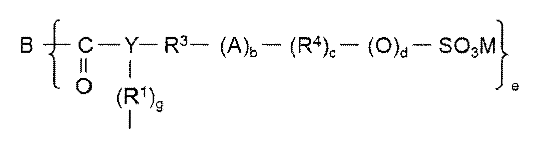 sein kann, wobei in diesem Falle a = 0 ist
R³ ein, unsubstituiertes oder mit einer funktionellen Gruppe substituiertes, geradkettiges, verzweigtes oder mindestens einen Cyclus aufweisendes Alkylen ist,
A eine Verknüpfungsgruppe darstellt,
R⁴ ein gegebenenfalls durch O, S, NH oder NR⁸ unterbrochenes und/oder funktionell substituiertes Alkylen bedeutet,
M Wasserstoff oder ein einwertiges Metallkation oder der entsprechende Anteil eines mehrwertigen Metallkations, vorzugsweise ausgewählt unter Alkali- und Erdalkalikationen, insbesondere unter Na, K, ½Ca, 1/2Mg, oder Ammonium ist, R⁵ und R⁶ unabhängig voneinander entweder unter Hydrolysebedingungen kondensierbare Reste oder substituiertes oder unsubstituiertes, geradkettiges, verzweigtes oder mindestens einen Cyclus aufweisendes Alkyl, Aryl, Arylalkyl, Alkylaryl oder Alkylarylalkyl sind, ausnahmsweise stattdessen aber auch ein entsprechendes Alkylen, Arylalkylen oder Alkylenaryl sein können,
R⁸ C₁-C₆-Alkylen oder ein (Meth)Acrylrest ist,
B Vinyl, 2-Allyl oder, im Falle von e > 1, ein organischer Rest mit e Vinylgruppen ist, die jeweils an eine in der geschweiften Klammer befindliche Gruppe gebunden vorliegen
Y ein Stickstoffatom, -O-CH=, -S-CH= oder-NH-CH= ist, wobei jeweils das Sauerstoffatom, das Schwefelatom bzw. die NH-Gruppe eine Bindung zur benachbarten C(O)-Gruppe aufweist,
a = 0 oder 1 ist
b = 0 oder 1 ist
c = 0 oder 1 ist
mit der Maßgabe, dass für die Kombination von Y gleich einem Stickstoffatom, b = 0 und c = 0 der Rest R³ ggf. substituiertes Ethylen ist, sowie der Maßgabe, dass für die Kombination von Y gleich einem Stickstoffatom, b = 0 und c = 1 der Rest R⁴ ein durch O, S, NH oder NR⁸ unterbrochenes und gegebenenfalls funktionell substituiertes Alkylen bedeutet,
d = 0 oder 1 ist, und
e = 1, 2 oder 3 ist
f = 0 oder 1 ist und
g = 0 oder 1 ist,
wobei dann, wenn f = 1 ist, a und g beide # 0 sind und
wobei dann, wenn f = 0 ist, zumindest einer der Reste R³ oder R⁴ einen funktionellen Substituenten trägt, der einen (Meth)Acrylrest oder eine saure Gruppe aufweist oder R⁴ ein mindestens durch S unterbrochenes Alkylen bedeutet.

**3.** Verbindung nach Anspruch 1 oder Anspruch 2, worin die Verknüpfungsgruppe A in der Formel (la) ausgewählt ist unter (gelesen von links nach rechts in der Formel la) C(O)NH, NHC(O), NR⁷C(O), C(O)O und OC(O), wobei R⁷ wie in Anspruch 1 definiert ist.

**4.** Verbindung nach einem der voranstehenden Ansprüche, worin mindestens einer der Reste R³ und R⁴ substituiert ist mit mindestens einer Hydroxygruppe und/oder mit einem Rest R⁹COOM und/oder mit einem Rest SO₃M. worin M wie in Anspruch 2 definiert ist, worin R⁹ eine chemische Bindung oder ein C₁-C₆-Alkylenrest ist und M Wasserstoff oder ein einwertiges Metallkation oder der entsprechende Anteil eines mehrwertigen Metallkations ist.

**5.** Verbindung nach einem der voranstehenden Ansprüche, worin f = 1 ist und g = 1 ist.

**6.** Verbindung nach einem der voranstehenden Ansprüche, worin f = 0 ist und g = 0 ist und a = 0 ist.

**7.** Verfahren zum Herstellen einer Verbindung mit der Formel (II) wie in Anspruch 2 definiert,
**dadurch gekennzeichnet, dass**
(a) eine Kohlenwasserstoffverbindung bereitgestellt wird, die mindestens zwei funktionelle Gruppen trägt, ausgewählt unter primären Aminen, sekundären Aminen, Hydroxygruppen und Thiolgruppen,
(b) eine erste der beiden funktionellen Gruppen mit gegebenenfalls aktivierter (Meth)Acrylsäure und die zweite der beiden funktionellen Gruppen mit einer gegebenenfalls aktivierten, zweiten, eine C=C-Doppelbindung aufweisenden Carbonsäure umgesetzt werden und
(c) im Anschluss an die genannte Reaktion eine sulfonat- oder sulfatgruppenhaltige Verbindung oder ein Sulfit an die C=C-Doppelbindung des mit der zweiten funktionellen Gruppe umgesetzten Carbonsäurerestes addiert wird, derart, dass an dem mit der ersten der beiden funktionellen Gruppen umgesetzten (Meth)Acrylrest eine solche Addition nicht stattfindet,
wobei es sich bei der zweiten eine C=C-Doppelbindung aufweisenden Carbonsäure um (Meth)Acrylsäure oder um eine andere doppelbindungshaltige Carbonsäure handeln kann.

**8.** Verfahren zum Herstellen einer Verbindung der Formel (II) wie in Anspruch 2 definiert, **dadurch gekennzeichnet, dass**
(a) eine Kohlenwasserstoffverbindung bereitgestellt wird, die mindestens einen Heteroring trägt, ausgewählt unter Oxacycyclopropyl, Azacyclopropyl, Thiocyclopropyl und einem cyclischen Carbonat,
(b) der Heteroring mit einem Sulfit oder einer sulfonat- oder sulfatgruppenhaltigen Verbindung umgesetzt wird und
(c) zumindest die dabei freiwerdende OH-, SH- bzw. NH₂-Gruppe mit gegebenenfalls aktivierter (Meth)Acrylsäure umgesetzt wird.

**9.** Verfahren nach einem der Anspruch 7 oder 8, **dadurch gekennzeichnet, dass** die sulfonat- oder sulfatgruppenhaltige Verbindung eine OH-, SH- oder NHR¹⁰-Gruppe mit R¹⁰ = Wasserstoff oder C₁-C₆-Alkyl, insbesondere eine SH-Gruppe aufweist.

**10.** Verfahren zum Herstellen einer Verbindung der Formel (II) wie in Anspruch 2 definiert, **dadurch gekennzeichnet, dass**
(a) eine Kohlenwasserstoffverbindung bereitgestellt wird, die eine Alkylaminogruppe oder eine Mercaptogruppe aufweist,
(b) ein Alkenylsulfonat mit der Alkylaminogruppe oder der Mercaptogruppe zur Reaktion gebracht wird und
(c) die in (b) entstandene sekundäre Aminogruppe mit gegebenenfalls aktivierter (Meth)acrylsäure umgesetzt wird.

**11.** Verfahren zum Herstellen einer Verbindung der Formel (II) wie in Anspruch 2 definiert, **dadurch gekennzeichnet, dass**
(a) eine organische Verbindung mit mindestens drei (Meth)Acrylgruppen bereitgestellt wird, und
(b) eine sulfonat- oder sulfatgruppenhaltige Verbindung oder ein Sulfit im Unterschuss an die C=C-Doppelbindung eines oder mehrerer der (Meth)Acrylreste addiert wird, derart, dass an mindestens einem (Meth)Acrylrest im Molekül eine solche Addition nicht stattfindet.

**12.** Verfahren nach einem der Ansprüche 7 bis 10, **dadurch gekennzeichnet, dass** die Kohlenwasserstoffverbindung ein Silan ist, das eine Si-C-gebundene Kohlenwasserstoffgruppe, vorzugsweise eine substituierte oder unsubstituierte, gegebenenfalls durch O, S oder NH unterbrochene oder eine Verknüpfungsgruppe enthaltende Alkylengruppe ist.

**13.** Verfahren zum Herstellen einer Verbindung wie in Anspruch 6 definiert, **dadurch gekennzeichnet, dass** eine organische Verbindung, die mindestens eine (Meth)Acrylrest und mindestens einen Alkenylcarboxylrest aufweist, mit einer sulfonat- oder sulfatgruppenhaltigen Verbindung oder einem Sulfit umgesetzt wird, dass diese(s) an die C=C-Doppelbindung des Alkenylcarboxylrestes addiert wird, derart, dass an dem (Meth)Acrylrest eine solche Addition nicht stattfindet, wobei es sich bei dem mindestens einen Alkenylcarboxylrest um einen zweiten (Meth)Acrylrest oder um den Rest einer anderen Alkenylcarbonsäure handeln kann.

**13.** Polymerisat, erhalten aus oder unter Verwendung mindestens einer Verbindung wie in einem der Ansprüche 1 bis 6 definiert durch Polymerisation mindestens eines Teils von deren (Meth)Acrylgruppen.

**14.** Verwendung eines Polymerisats nach Anspruch 13 für dentale Zwecke, insbesondere als Dentaladhäsiv.
